# EUROPEAN PATENT APPLICATION

(11) **EP 4 691 462 A1**
(43) Date of publication of application: **11.02.2026**
(21) Application number: 24777814.5
(22) Date of filing: 20.03.2024
(51) Int. Cl.: A61K 31/403, A61K 31/357, A61P 1/16

(54) **SILYBIN-CONTAINING COMPOSITION**

(30) Priority: 27.03.2023 CN 202310307875
(71) Applicant: Tianjin Tasly Sants Pharmaceutical Co., Ltd., Tianjin 300410 (CN); China Pharmaceutical University, Nanjing, Jiangsu 210009 (CN)
(72) Inventor: HAO, Haiping, Nanjing, Jiangsu 210009 (CN); WANG, Hong, Nanjing, Jiangsu 210009 (CN); CHEN, An, Nanjing, Jiangsu 210009 (CN); ZHU, Xiaochai, Nanjing, Jiangsu 210009 (CN); WANG, Guangji, Nanjing, Jiangsu 210009 (CN); MA, Xiaohui, Huaian, Jiangsu 223003 (CN); ZHANG, Jun, Huaian, Jiangsu 223003 (CN); XIN, Lingyi, Huaian, Jiangsu 223003 (CN); CAI, Jinyong, Huaian, Jiangsu 223003 (CN); ZHOU, Shuiping, Huaian, Jiangsu 223003 (CN); TANG, Hai, Huaian, Jiangsu 223003 (CN); RONG, Hua, Huaian, Jiangsu 223003 (CN); ZHAO, Meiling, Huaian, Jiangsu 223003 (CN)
(74) Representative: Gulde & Partner
(86) International application number: PCT/CN2024/082603
(87) International publication number: WO 2024/199025

(57) **Abstract**

The present invention relates to the use of a silybin-containing composition in the treatment of hepatic fibrosis. A silybin-containing composition contains silybin and carvedilol as active ingredients. The silybin composition disclosed in the present invention can significantly down-regulate the expression levels of Col1a1, Col1a2(Col3a1) and Mmp2 mRNA, and can effectively ameliorate various chronic liver disease such as chemical liver damage, cholestatic liver disease and non-alcoholic fatty liver, and the degree of liver fibrosis caused by said diseases.

## Description

### TECHNICAL FIELD

The present invention relates to the technical field of pharmaceutics, specifically relates to the use of a silybin-containing composition in the treatment of hepatic fibrosis.

### BACKGROUND ART

Silybum marianum (L.) Gaertn., is a dry mature fruit of Asteraceae plant Silybum marianum. It tastes bitter and cool, and has the effects of clearing heat and detoxifying, soothing liver and promoting gallbladder function. It has good therapeutic effects on acute and chronic hepatitis, liver cirrhosis, fatty liver, and the like in modern clinical application. Its active ingredient silybin (SB) is one of the flavonoid extracts with the highest market share in the world (Bhattacharya, S. Phytotherapeutic Properties of Milk Thistl-e Seeds: An Overview. J. Adv. Pharm. Educ. Res. 2011, 1, 69-79.).

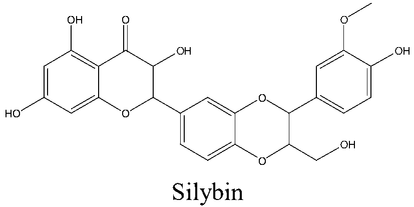

Silybin can reduce ALT, AST and other liver enzyme levels to prevent cell escape and loss of liver cell membrane functional integrity. In addition, silybin can also protect hepatocytes by reducing CH, TG and LDL levels. Silybin can trigger a variety of antioxidant enzymes and stimulate Nrf2 pathway to reduce oxidative stress. Silybin proved its anti-inflammatory effect in NAFLD and NASH from the perspective of inhibiting the release of cytokines, which can significantly reduce the levels of pro-inflammatory cytokines TNF-α, IL-6, IL-1β and IL-12β. CN202111261969.0 discloses a novel preparation of high-purity silybin meglumine medicament, a preparation method thereof and application in preventing and treating acute, dangerous and severe liver failure. CN202210427370.8 discloses a method for preparing silybin derivatives and the effect of silybin derivatives on reducing oxidative damage. Silybin has definite protective effect on liver, but silybin has weak protective effect on hepatic fibrosis. Due to poor water solubility of silybin, most of the current researches are on the treatment of hepatic fibrosis by silybin injection, which can only reduce serum hepatic fibrosis indexes, α-SMA protein, TGF-β1 protein, and the like.

Hepatic fibrosis is the excessive deposition of diffuse extracellular matrix (especially collagen) in liver caused by a variety of chronic liver diseases. It is mainly manifested by increased transcription of fibrosis-promoting molecules such as α-SMA protein, type I and III collagen and matrix metalloproteinase inhibitors, resulting in a large number of fibrous and non-fibrous matrix proteins. Persistent or repeated inflammatory necrosis of liver parenchyma cells caused by chronic liver disease causes a large amount of proliferation of fibrous connective tissue, and its degradation activity is relatively or absolutely insufficient, resulting in a dynamic imbalance between the synthesis of various extracellular matrix fibers and the degradation of course. Therefore, a large amount of extracellular matrix deposits form hepatic fibrosis. Hepatic fibrosis is a reversible course in the continuous development of chronic liver disease into cirrhosis. Recent basic and clinical studies have shown that effective etiological treatment can directly inhibit extracellular matrix synthesis and promote degradation, thus restoring the "net effect" of various extracellular matrix fiber synthesis and degradation of course and achieving dynamic balance. Activation of Hepatic Stellate Cells (HSCs) is the central link of hepatic fibrosis. Normal liver HSCs mainly store vitamin A, which causes hepatocyte apoptosis after liver injury. Apoptosis bodies activate Kuffer cells and platelets to secrete cytokines and chemokines such as transforming growth factor β(TGF-β), platelet-derived growth factor (PDGF). Damaged hepatocytes and endothelial cells release oxygen free radicals and inflammatory factors, and jointly mediate resting HSCs to synthesize a large number of ECM into activated myofibroblasts. As silybin has definite liver protection effect but weak anti-hepatic fibrosis effect, a reasonable combination of chemical drugs acting on different targets of HSCs activation and silybin will probably make a breakthrough in reversing treatment of hepatic fibrosis caused by liver injury.

### SUMMARY OF THE INVENTION

The present invention provides a silybin-containing composition, and provides the use of the composition in the treatment of hepatic fibrosis.

The present invention provides the following technical solutions.

A silybin-containing composition, comprising silybin and carvedilol as active ingredients.

A silybin-containing composition, comprising a) silybin and carvedilol as active ingredients; and b) a pharmaceutically acceptable excipient suitable for preparaing a solid oral dosage form; wherein the mass ratio of silybin and carvedilol is 0.1 to 200: 1.

The silybin-containing composition of the present invention, wherein the carvedilol includes the pharmaceutically acceptable salt formed with organic acid or inorganic acid. The organic acid includes but is not limited to oxalic acid, fumaric acid, benzoic acid and mandelic acid; and the inorganic acid includes but is not limited to phosphoric acid.

In a further embodiment, the present invention further discloses the following silybin-containing composition, with the specific composition shown below.

A silybin-containing composition, comprising (a) silybin, and (b) a non-steroidal anti-inflammatory drug as active ingredients; wherein the non-steroidal anti-inflammatory drug is selected from aceclofenac or clobetasol propionate.

A silybin-containing composition, comprising (a) silybin, and (b) an angiotensin inhibitor as active ingredients, wherein the angiotensin inhibitor is valsartan.

A silybin-containing composition, comprising (a) silybin, and (b) a calcium antagonist as active ingredients, wherein the calcium antagonist is selected from nicardipine, manidipine, amlodipine or cilnidipine.

A silybin-containing composition, comprising (a) silybin, and (b) a diuretic as active ingredients; wherein the diuretic is selected from tamsulosin or D-mannitol.

A silybin-containing composition, comprising (a) silybin, and (b) a glucocorticoid drug as active ingredients; wherein the glucocorticoid drug is budesonide.

In addition, when the above compounds constituting the composition of the present invention with silybin, including carvedilol, non-steroidal anti-inflammatory drugs, angiotensin inhibitors, calcium antagonists, diuretics and glucocorticoid drugs, have asymmetric carbon atoms, also include their optical isomers and mixtures of their isomers. In particular, the composition of the present invention further comprises silybin and one or more of the above compounds, including prodrugs formed by carvedilol, non-steroidal anti-inflammatory drugs, angiotensin inhibitors, calcium antagonists, diuretics and glucocorticoids.

In some embodiments, the present invention discloses the use of a silybin-containing composition, including a composition containing silybin and carvedilol, and a composition formed by silybin and non-steroidal anti-inflammatory drugs, angiotensin inhibitors, calcium antagonists, diuretics and glucocorticoids in the treatment of hepatic fibrosis, and the use of the composition in the treatment of chronic liver diseases.

Furthermore, the use is the use in the treatment of chronic liver diseases and the hepatic fibrosis caused thereby.

Furthermore, the use is the use in the treatment of chronic liver diseases such as chemical liver damage, cholestatic liver disease, non-alcoholic steatohepatitis (NASH) and the like and the hepatic fibrosis caused thereby.

According to the present invention, the combination of silybin and carvedilol shows better effect than each single dose. Therefore, silybin and carvedilol can be administered in the form of a mixed dose. The administration route of the silybin and carvedilol composition disclosed in the present invention is generally oral administration. Therefore, the two kinds of medicaments can be prepared into separate individual unit administration forms or mixed physical single unit administration forms. The unit administration forms include, for example, powder, granule, tablet, capsule, and the like. The bulk drug can be mixed with excipients, diluents, and the like. The bulk drug can be prepared by common preparation techniques as described below. The solid oral dosage form of the present invention includes additives commonly used in the dosage form, including pharmaceutical excipients commonly used in tablet or capsule preparations. These substances include, but are not limited to, disintegrants, binders, lubricants, glidants, stabilizers, excipients or diluents, solubilizers, and the like. The silybin- containing composition is in the form of tablets or capsules.

The above preparation can be made of excipient (organic excipients, such as sugar derivatives such as lactose, sucrose, glucose, mannitol, sorbitol, and the like; starch derivatives such as corn starch, potato starch, alpha starch, dextrin, and the like; cellulose derivatives such as microcrystalline cellulose; gum arabic; glucan; pullulan; and inorganic excipients, such as silicate derivatives such as light silicic anhydride, synthetic aluminum silicate, calcium silicate, aluminosilicate, and the like; phosphate such as calcium hydrogen phosphate; carbonate such as calcium carbonate; sulfates such as calcium sulfate), lubricants (such as metal stearate such as stearic acid, calcium stearate, magnesium stearate; talc powder; waxes such as beeswax and whale wax; boric acid; adipic acid; sulfates such as sodium sulfate and the like; ethylene glycol; fumaric acid; sodium benzoate; DL-leucine; lauryl sulfate such as sodium lauryl sulfate and lauryl sulfate soap; silicic acids such as silicic anhydride and silicic acid hydrate; as well as starch derivative described above), binder (such as hydroxypropyl cellulose, hydroxypropyl methylcellulose, polyvinylpyrrolidone, polyethylene glycol and the same compound as the above excipients), disintegrant (such as cellulose derivatives such as low substituted hydroxypropyl cellulose, carboxymethyl cellulose, calcium carboxymethyl cellulose, internally crosslinked sodium carboxymethyl cellulose, and the like; chemically modified starch cellulose such as carboxymethyl starch, carboxymethyl starch sodium, crosslinked polyvinylpyrrolidone, and the like; the starch derivatives mentioned above), emulsifiers (such as bentonite, gum clay such as V-gum, and the like; metal hydroxides such as magnesium hydroxide and aluminum hydroxide; anionic surfactants such as sodium lauryl sulfate and calcium stearate; cationic surfactants such as benzalkonium chloride; and nonionic surfactants such as polyoxyethylene alkyl ethers, polyoxyethylene sorbitan fatty acid esters, sucrose fatty acid esters, and phosphorus esters such as soybean phospholipids), stabilizers (such as parabens such as methyl paraben, propyl paraben, and the like; alcohols such as chlorobutanol, ethanol, phenylethanol and the like; benzalkonium chloride; phenols such as phenol and cresol; dehydroacetic acid; and sorbic acid), flavor correctives (such as commonly used sweeteners, sours, spices and the like), diluents and other additives by commonly known methods.

The combination of silybin with a dosage range of about 0.2 to 8000 mg and carvedilol with a dosage range of about 2 to 40 mg is suitable for more effective treatment of hepatic fibrosis. Particularlly suitable dosage ranges are about 1.5 to 3000 mg silybin and about 3 to 20 mg carvedilol. More preferable unit doses are about 3 to 1000 mg silybin and about 3 to 10 mg carvedilol. More preferable unit doses are about 15 to 500 mg silybin and about 3 to 10 mg carvedilol. More preferable unit doses are about 30 to 500 mg silybin and about 3 to 10 mg carvedilol. More preferable unit doses are about 90 to 500 mg silybin and about 3 to 10 mg carvedilol. More preferable unit doses are about 120 to 500 mg silybin and about 3 to 10 mg carvedilol. Most preferable unit doses are about 150 to 500 mg silybin and about 3 to 10 mg carvedilol.

The combination of silybin and carvedilol disclosed by the present invention can significantly down-regulate the expression levels of Col1a1, Col1a2(Col3a1) mRNA, and can effectively ameliorate the degree of liver fibrosis pathological changes in various hepatic fibrosis models. The above drugs are preferably used in warm-blooded animals, more preferably in humans.

### BRIEF DESCRIPTION OF DRAWINGS

Figure 1 shows the establishment and validation of HSCs activation model based on *Col1a1-*Luci-reporter luciferase; Compared with the blank vehicle group, ***p < 0.001; Compared with the control group, ns>0.05, ### P <0.001;
Figure 2 shows the investigation of silybin on activation of HSCs; Compared with the blank vehicle group, ***p < 0.001; Compared with the control group, ns>0.05;
Figure 3 shows the screening of silybin composition inhibiting HSCs activation, wherein:
   Figure 3-1 shows the analysis of the expression level of *Col1a1-*Luci-reporter luciferase in activated HSCs cells by combination of a class of antihypertensive compounds (represented by carvedilol) and silybin;
   Figure 3-2 shows the analysis of expression level of *Col1a1-*Luci-reporter luciferase in activated HSCs cells by combination of other compounds and silybin; Compared with the blank vehicle group, ***p < 0.001; Compared with the control group, ns>0.05, #P < 0.05, ##P < 0.01, ### p < 0.001;
Figure 4 shows the validation of inhibitory effect of silybin combined with carvedilol on activation of Primary hepatic stellate cells (pHSCs). PHSCs was isolated and extracted from mice. Carvedilol, silybin were given separately or simultaneously, and mRNA levels of Col1a1 and Col1a2 were analyzed. ns>0.05, **P < 0.01, ***P < 0.001;
Figure 5 shows the administration ratio analysis of silybin and carvedilol on HSC activation inhibition, wherein:
   Figure 5-1 shows the inhibitory effect on HSCs activation of the concentration ratio with the administration concentration of carvedilol as fixed 10 µM, and the ratio of the two as 6:1; 4:1; 2:1; 1:1; 1:2; 1:4; 1:6; 1:8;
   Figure 5-2 shows the inhibitory effect on HSCs activation of the concentration ratio with the administration concentration of silybin as fixed 10 µM, and the ratio of the two as 6:1; 4:1; 2:1; 1:2; 1:4; 1:6; 1:8; Compared with the blank vehicle group, ***p < 0.001; Compared with the control group, ns>0.05, ##P < 0.01, ### p < 0.001;
Figure 6 shows the effect of silybin (100 mg/kg) combined with carvedilol (2 mg/kg) on CCl₄-induced hepatic fibrosis, wherein:
   Figure 6-1 shows the expression level analysis of liver *Col1a1* and *Col1a2* mRNA;
   Figure 6-2 shows the liver HE, Sirius Red, Masson staining analysis;
   Figure 6-3 shows the comparison of liver collagen volume fraction (CVF) of mice in each group, ns>0.05, *P < 0.05, **P <0.01, ***P <0.001;
Figure 7 shows the effect of silybin (100 mg/kg) combined with carvedilol (2 mg/kg) on BDL-induced hepatic fibrosis, wherein:
   Figure 7-1 shows the liver *Col1a1, Col1a2* mRNA expression level analysis;
   Figure 7-2 shows the liver HE, Sirius Red, Masson staining analysis;
   Figure 7-3 shows the comparison of liver collagen volume fraction (CVF) of mice in each group, ns>0.05, *P < 0.05, **P <0.01, ***P <0.001.
Figure 8 shows the effect of silybin (75,100 mg/kg) combined with carvedilol (2 mg/kg) on NASH and hepatic fibrosis induced by MCD diet, wherein:
   Figure 8-1 shows the liver *Col1a1, Col1a2, Col3a1* mRNA and Mmp2 expression level analysis;
   Figure 8-2 shows the liver HE, Sirius Red, Masson staining analysis;
   Figure 8-3 shows the comparison of liver collagen volume fraction (CVF) and lipid vacuole area in each group of mice, compared with control group (MCS), ns>0.05, *P < 0.05, **P <0.01, ***P <0.001, compared with MCD group, ns>0.05, #P < 0.05, ##P <0.01, ### p < 0.001;
Figure 9 shows the dose-dependent effect of silybin and carvedilol composition with fixed ratio on CCl₄-induced hepatic fibrosis, wherein:
   Figure 9-1 shows the liver *Col1a1* and *Col1a2* mRNA expression level analysis
   Figure 9-2 shows the liver HE, Sirius Red, Masson staining analysis;
   Figure 9-3 shows the comparison of liver collagen volume fraction (CVF) of mice in each group; compared with the control group, ns>0.05, *P < 0.05, **P <0.01, ***P <0.001, compared with the model group, ns>0.05, #P < 0.05, ##P <0.01, ### P < 0.001;
Figure 10 shows the dose-dependent effect of silybin and carvedilol composition with fixed ratio on BDL-induced hepatic fibrosis, wherein:
   Figure 10-1 shows the liver *Col1a1, Col1a2* mRNA expression level analysis;
   Figure 10-2 shows the liver HE, Sirius Red, Masson staining analysis;
   Figure 10-3 shows the comparison of the liver collagen volume fraction (CVF) of mice in each group; compared with the control group, ns>0.05, *P < 0.05, **P <0.01, ***P <0.001, compared with the model group, ns>0.05, #P < 0.05, ##P <0.01, ### P < 0.001.

### DETAILED DESCRIPTION OF THE INVENTION

The present invention is further illustrated by the following examples, but not by way of limitation.

The following experimental examples investigates the effectiveness of carvedilol combined with silybin on hepatic fibrosis.

The carvedilol of the present invention has the CAS Number of 72956-09-3, and the structural formula of the following:

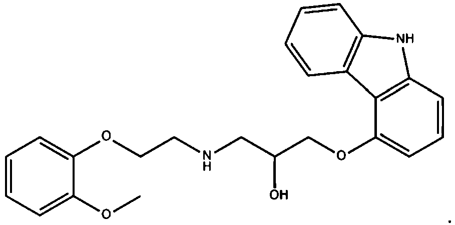

### Experimental Example 1 Establishment and validation of HSCs activation model based on Col1a1-Luci-reporter luciferase

### 1 Experimental Materials

The human hepatic stellate cell line LX-2 cell line used in the present invention was purchased from Wuhan Purcell Life Science and Technology Co., Ltd. and was conventionally cultured at 37 °C and 5%. The culture medium was DMEM (Gibco) containing 10% fetal bovine serum. Fetal bovine serum was purchased from Hyclone (Logan, Utah, USA). Trypsin was available from Amersco (Solon, Ohio, USA). OptiMEM medium was purchased from Gibco (California, USA). Lipofectamine 3000 liposome transfection reagent was purchased from Invitrogen (Waltham, USA). Pirfenidone used in the present invention was purchased from MedChemExpress (New Jersey, USA). DuoLiteTM Luciferase Assay System reporter gene kit was purchased from Nanjing Vazyme Biotech Co., Ltd. (Nanjing, China). pEZX-FR03-hygro empty plasmid and pEZX-FR03-hygro-COL1A1-WT plasmid were constructed from Beijing Tsingke Biotech Co., Ltd..

### 2 Experimental Methods

### 2.1 LX-2 Cell Line Culture and Transfection

LX-2 cells were inoculated into 96-well plates and cultured to a suitable density. Then, Lipofectamine 3000 liposome transfection reagent was used to prepare transfection system. Each well was transfected with 0.1 µg plasmid, of which the Empty vector group was transfected with pEZX-FR03-hygro empty plasmid, and the Control group and other administration groups were transfected with pEZX-FR03-hygro-COL1A1-WT plasmid.

### 2.2 LX-2 Cell Administration

The LX-2 cell line was transfected with plasmid for 6 hours, and then the drug-containing serum-free medium containing 2.5 mM, 5 mM, and 10 mM anti-fibrosis agent Pirfenidone was replaced according to groups for incubation for 12 hours, and then tested by the Duo-LitTM Luciferase Assay System kit.

### 2.3 Duo-LiteTM Luciferase Assay System Kit Detection

The cell culture plate to be tested was taken out into the incubator, and left at room temperature for 30 min to allow the temperature of the culture plate to be balanced to room temperature. Firefly luciferase activity was detected. The original culture medium was discarded, and added with 75 µl PBS, then added with Duo-Lite Luciferase detection reagent with the same volume as PBS, and was mixed evenly. The system was left at room temperature for 10 min to detect firefly luciferase luminescence.

### 3 Experimental Results

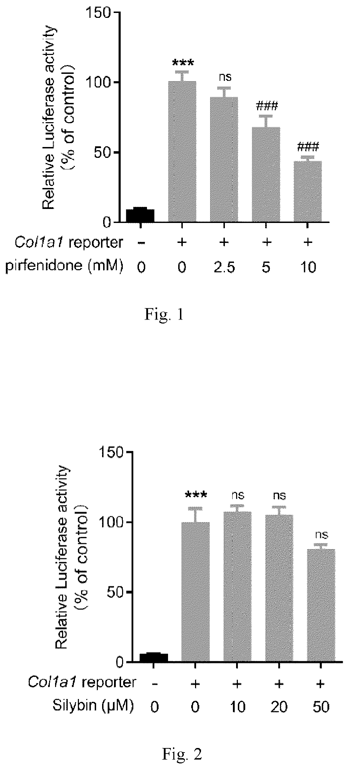

As shown in Figure 1, in LX-2 cells cultured in vitro, control cells transfected with *Col1a1* reporter gene stably and highly expressed *Col1a1*-Luci-reporter luciferase based on the activated state characteristics of stellate cells. The experimental group was given 2.5 mM, 5 mM, and 10 mM anti-fibrosis agent Pirfenidone, of which 5 mM, 10 mM concentration of Pirfenidone significantly decreased the expression level of luciferase, showing a clear dose-dependence. A HSCs activation model based on *Coll a1*-Luci-reporter luciferase was constructed and verified by the positive drug Pirfenidone.

### Experimental Example 2 Investigation on Activation of HSCs by Silybin

### 1 Experimental Materials

Silybin used in the present invention was purchased from Nanjing Zelang Biological Technology Co., Ltd..

The rest of the experimental materials were the same as those in Experimental Example 1.

### 2 Experimental Methods

### 2.1 LX-2 Cell Line Culture and Transfection

The specific method was the same as part 2.1 of Experimental Example 1.

### 2.2 LX-2 Cell Administration

The LX-2 cell line was transfected with plasmid for 6 hours, and then the drug-containing serum-free medium containing 10 µM, 20 µM, 50 µM Silybin was replaced according to groups for incubation for 12 hours, and then tested by the Duo-LitTM Luciferase Assay System kit.

### 2.3 Duo-LiteTM Luciferase Assay System Kit Detection

The specific method was the same as part 2.3 of Experimental Example 1.

### 3 Experimental Results

As shown in Figure 2, the efficacy of 10 µM, 20 µM and 50 µM silybin was verified on HSCs activation model based on *Coll a1*-Luci-reporter luciferase using LX-2 cells. The results showed that 50 µM silybin and below had no significant inhibitory effect on activation of stellate cells in vitro.

### Experimental Example 3 Screening of Silybin Composition for Inhibiting HSCs Activati on

### 1 Experimental Materials

The drugs involved in the screening (rofecoxib, pranoprofen, meloxicam, hydrocortisone acetate, sulindac, fluocinonide, loxoprofen sodium, desonide, and the like) were all from FDA listed drug bank, MCE-FDA listed drug bank-HY-L1022, PerkinElmer high-throughput drug screening workstation of China Pharmaceutical University.

Other experimental materials were the same as those in Experimental Examples 1 and 2.

### 2 Experimental Methods

### 2.1 LX-2 Cell Line Culture and Transfection

The specific method was the same as part 2.1 of Experimental Example 1.

### 2.2 LX-2 Cell Administration

The LX-2 cell line was transfected with plasmid for 6 hours, and then the drug-containing serum-free medium containing 10 µM silybin, 10 µM drug to be screened, 20 µM silybin, 20 µM drug to be screened at a mixed ratio of 1:1 was replaced according to groups for incubation for 12 hours, and then tested by the Duo-LitTM Luciferase Assay System kit.

### 2.3 Duo-LiteTM Luciferase Assay System Kit Detection

The specific method was the same as part 2.3 of Experimental Example 1.

### 2.4 Analysis of Compound Joint Action Index

Cell activation inhibition rate% = (Control Group Firefly- Administration Group Firefly)/ Control Group Firefly ×100%. The CI value was obtained by analysis using micro-use CompuSyn software. The CI value of 0.90<CI<1.10 indicates that the two have additive effect, CI<0.90 indicates that the two have synergistic effect, and the smaller the CI value, the stronger the synergistic effect.

### 3 Experimental Results

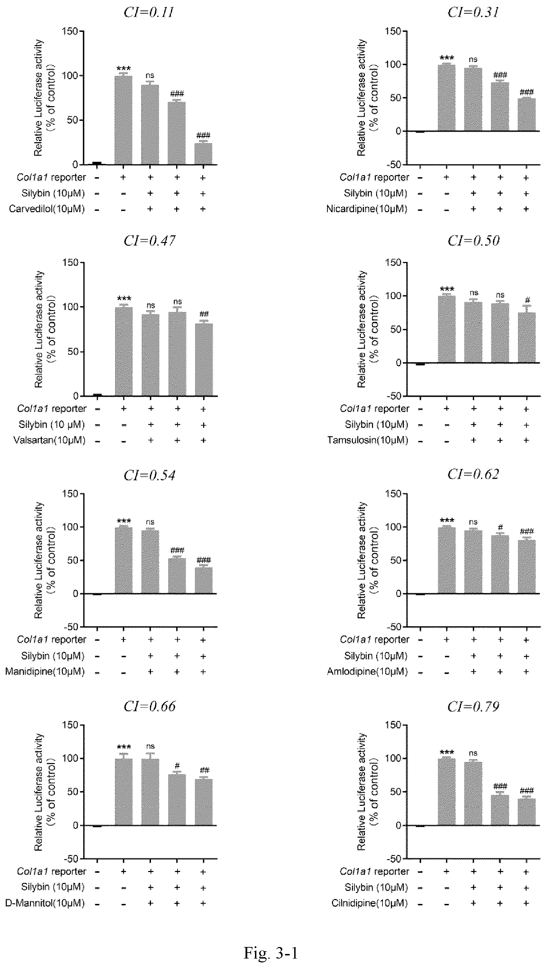

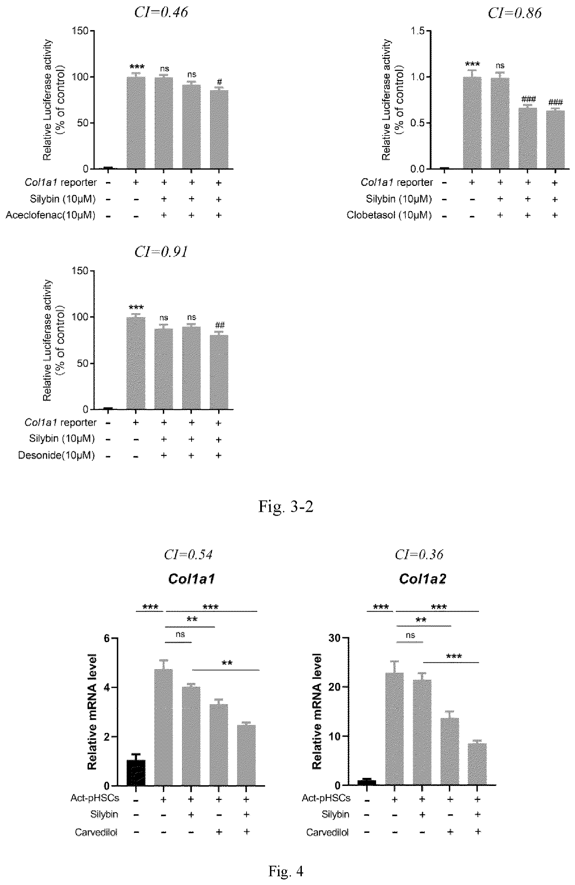

As shown in Figures 3-1 and 3-2, the results showed that silybin has synergistic effects with the following drugs: carvedilol (CI=0.11), nicardipine (CI=0.31), valsartan (CI=0.47), tamsulosin (CI=0.50), manidipine (CI=0.54), amlodipine (CI=0.62), D-mannitol (CI=0.66), cilnidipine (CI=0.79), aceclofenac (CI=0.46), clobetasol propionate (ci = 0.86); and it has additive effect with budesonide (CI=0.91). Among them, silybin and carvedilol have the strongest synergistic effect.
Note: Screening of Silybin Compositions for Inhibiting HSCs Activation

Drugs selected from FDA's drug list include rofecoxib, pranoprofen, meloxicam, hydrocortisone acetate, ramipril, irbesartan, lacidipine, sulindac, fluocinonide, loxoprofen sodium, budesonide, idebenone amodiaquine hydrochloride, azelaic acid, clobetasol propionate, chlorofluorocarbon, phenylthioamine, flumetasone, mannitol, fluoroniac acid, 5-aminosalicylic acid, ketoprofen, bromofenac (sodium hydrate), ipratropium, valdecoxib, troglitazone, L-ascorbic acid, imatinib mesylate, rigfenib hydrochloride, punitinib, carbotinib malate, quinapril hydrochloride, benazepril hydrochloride, perindopril tert-butylamine, cilazapril hydrate, zofenpril, imidapril hydrochloride, mosinopril, teminopril hydrochloride, captopril, enalapril maleate, losartan, candesartan, valsartan, ipratan, olmesartan medoxomil, femasatan, telmisartan, olmesartan, azilsartan, nisoldipine, benidipine hydrochloride, ifoldipine hydrochloride monoethanol salt, nitrendipine hydrochloride, manidipine hydrochloride, clonidipine, nifedipine, cilnidipine, nimodipine, amlodipine, nicardipine hydrochloride, nilvadipine, felodipine, verapamil hydrochloride, narizine, aliskiren, carvedilol, spironolactone, fosinopril sodium, enalaprilat, triamcinolone, zorimide, troxipide, tiotropium bromide, flurbiprofen, roflumilast, telarone hydrochloride, budesonide, etoricoxib, sunitinib, homoharringtonine, zofenopril calcium, amlexanox, salmeterol loxacin naphthoate, lenvatinib, aceclofenac, nitinib, apatinib, ruxolitinib, gefitinib, benzdamine hydrochloride, regofinib, firocoxib, dimethyl fumarate, beclomethasone dipropionate, mometasone, ganciclovir, Articaine hydrochloride, Sapropterin hydrochloride, Dextromethorphan (hydrobromide), riluzole, Avanafil, clenbuterol hydrochloride, dapoxetine hydrochloride, naltrexone hydrochloride, sulfanilamide, candesartan cilexetil, vardenafil hydrochloride, indacaterol maleate, cyclohexamethylenetetramine, desmopressin acetate, Calcium Fosfomycin, tirofiban (monohydrate hydrochloride), betamethasone dipropionate, daperazole hydrochloride, parecoxib, isoniazid, telaprevir, sulfaisoxazole, pyrazinamide, methylprednisolone, Sodium cromoglycate, sodium rezinade, metoprolol succinate, atenolol, acerbinol (hydrochloride), betaxolol, labetalol hydrochloride, arotinolol, propranolol hydrochloride, (S)-timolol maleate, phentolamine mesylate, tolazoline hydrochloride, prazosin hydrochloride, tamsulosin, bisoprolol hemifumarate, esmolol hydrochloride, sotalol hydrochloride, cartolol hydrochloride, alpolol (hydrochloride), phenoxybenzamine acid, yohimbine, Rauwolscine hydrochloride, icotinib, fedamycin, valnemulin, lamivudine, pulesaf, azaphen, cefoperazone, tegafur, difluoropregnane, sulbactam, citric acid, trimetinib, estrone sulfate piperazine, alprostadil, moclobemide, capecitabine, phentolamine, sulindac, sodium gluconate, acetylglutamine, bromhexine, sevelamer, dimethomorph, L-epinephrine, melphalanx, nitrogen mustard, hydroxyfasudil, Avastin, irinotecan, sulfamidine, cobitinib, tadireline, diiodoquinoline, ropinirole, deoxycholic acid, taconazole, prostaglandin E2, clefibrate, loxoprofen, bugattinib, Pentostatin, medroxyprogesterone, albendazole, vitamin B1, sodium phosphophenytoin, fusidic acid, natamycin, dacarbazine, sodium docusate, balofloxacin, suramin, methimazole, epirubicin, mitoxantrone, bosentan, lesinurad, Azatadine, tolazurin, donipenem, vorazozan fumarate, minapran, venlafaxine, dantrolene, tipyrimidine, palonosetron, Fenspiride, isotretinoin, fenofibrate acid, nedolomib sodium, lafutidine, flufenozine, fluticasone, cabergoline, carmofur, crocin G, tenofovir alafenamide, Drospirenone, lidocaine, mianserin, pyridostigmine, sodium salicylate, cefotaxime, sulfamethoxazole, Vinpocetine, daptomycin, doxycycline, phylloquinone, lapatinib, irinotecan, pravastatin, L-arginine, nimorazole, fluconazole, perphenazine, dihydroxy-theophylline, pimoxifloxacin, dapsone, temulin, risedronic acid, olanzapine, levobupivacaine, lovastatin, fluvastatin, teicoplanin, pheniramate, pimecillin, iguratimod, rifabutin, berberine, taurodeoxycholic acid, progesterone, hexamethylmelamine, levodopa, Duloxetine, galantamine, tinidazole, tolvaptan, guanidine thiocyanate, artemether, ivabradine, mosapride, tadanafil, remitiam, bosentan, trifluorouridine, dechlorazine, tamibarotene, gemcitabine, phenoxybenzamine, 3,4-diaminopyridine, primaquine, olopatadine, ethylamine citrate, pancuronium, cefmetazole, carbamazepine, tezoxamine, ipratropium bromide, Aripiprazole, prucalopride, diclofenac sodium, aminoglutethimide, clotrimazole, tranexamic acid, hydrochlorothiazide, ibuprofen, rifampicin, moxifloxacin, piroxicam, fludarabine, delamanid, levodropropizine, Rucaparib, granisetron, methacholine acetate, ribavirin, neomycin, Levonorgestrel, clozapine, febuxostat, tranilast, solifenacin, valganciclovir, ketotifen, minapran, daphnetin, lenalidomide, ondansetron, procainamide, fexofenadine, fenofibrate, prulifloxacin, sulfamethazine, bezafibrate, sumatriptan, levetiracetam, dozosamine, retapamulin, difenidol, glibenclamide, acetazolamide, hexamine hydrochloride, irbesartan, 9-hydroxyrisperidone, Ciprofibrate, Glimepiride, cefdinir, decitabine, vecuronium, ticlopidine, aspirin, haloperidol, gestodene, trimethyldiketone, fluorouridine, estradiol, rezagiline, nitrofurantoin, ezetimibe, acarbose, 5-fluorouracil, mexiletine, trokosovar, voglibose, leflunomide, Rivastigmine, Acitretin, promethazine, oxazine, glucosamine, isoniazid, saframide, prazosin, histamine, ethambutol, cefmenondox, Diltiazem, lincomycin, budoxifen, cidofovir, aminotoluic acid, tropisetron, tizanidine, scopolamine, etoposide, folinic acid, nalfenavir, chlorthalidone, carbidopa, finasteride citrate, salicylic acid, ubenimex, probenecid, enoxacin, anagrel, encidipine, levosulpiride, quinine, nicotine, heparin, penicillamine, neomycin, itraphylline, propofol, bipyrimidine, methoxysarin, sodium nitroprusside, etapenem sodium, omidazole, paramivir trihydrate, zanamivir, titanium dioxide, glutamic acid monosodium salt, L-omidine, 397 samples in total.

Among them, the combination with silybin to inhibit HSCs activation has synergistic or additive effects:
Synergistic: carvedilol (CI=0.11), nicardipine(CI=0.31), valsartan (CI=0.47), tamsulosin(CI=0.50), manidipine(CI=0.54), amlodipine(CI=0.62), D-mannitol(CI=0.66), cilnidipine(CI=0.79), aceclofenac (CI=0.46), clobetasol propionate(CI=0.86)
Additive: budesonide(*CI=0.91*)

**Table 1 Average CI Value Interval and Interaction Evaluation of Compound Combination**

| ***CI* Value Range** | **Interaction Evaluation** | **Symbol** |
|---|---|---|
| *CI_{wt}*<0.1 | Very Strong Synergy | + + + + + |
| 0.1<*CI_{wt}*<0.3 | Strong Synergy | + + + + |
| 0.3<*CI_{wt}*<0.7 | Slightly Strong Synergy | + + + |
| 0.7*<CI_{wt}*<0.85 | Moderate Synergy | + + |
| 0.85<*CI_{wt}*<0.90 | Weak Synergy | + |
| 0.90*<CI_{wt}*<1.10 | Addition | ± |
| 1.10<*CI_{wt}*<1.20 | Slight Antagonism | - |
| 1.20<*CI_{wt}*<1.45 | Moderate Antagonism | - - |
| 1.45*<CI_{wt}*<3.3 | Slighly Strong Antagonism | - - - |
| 3.3*<CI_{wt}*<10 | Strong Antagonism | - - - - |
| 10<*CI_{wt}* | Very Strong Antagonism | - - - - - |

| | | |
|---|---|---|
| Note: *NaN:* No Number | | |

### Experimental Example 4 Verification of Inhibitory Effect of Carvedilol Combined with Silybin on Activation of p-HSCs

### 1 Experimental Materials

Male C57BL/6J mice, 8 weeks old and 20-24g weight, were purchased from Beijing Vital River Animal Technology Co., Ltd.. Heparin sodium, EGTA, collagenase type IV, PronaseE, Dnase I were purchased from Sigma-Aldrich. Nycodenz was purchased from Axis-Shield/Alere Technologies AS. RNA extraction kit (RNAiso Plus reagent, Cat# 9109) was purchased from Takara (Japan). HiScript Q RT SuperMix for qPCR, ChamQ SYBR qPCR Master Mix was purchased from Vazyme (Cat# Q311-02/03, Nanjing, China). Carvedilol was purchased from MedChemExpress (New Jersey, USA).

The rest of the experimental materials were the same as those in Experimental Example 2.

### 2 Experimental Methods

### 2.1 Extraction of Mouse Primary Hepatic Stellate Cells (p-HSCs)

The mice were weighed and anesthetized by intraperitoneal injection of 5% chloral hydrate (0.1 ml/10 g). After anesthesia, the mice were sprayed with alcohol for disinfection and then fixed in place. The abdominal area was disinfected with an alcohol cotton ball, and the abdominal cavity was opened with scissors. The intestines were gently moved aside to expose the portal vein and inferior vena cava. The portal vein was isolated, and a suture was pre-placed beneath it. A cannula was inserted into the portal vein, and the suture was tied. The lower end of the inferior vena cava was cut open, and the thoracic segment of the inferior vena cava was clamped. Perfusion was initiated with pre-warmed EGTA solution, Pronase-E solution, and Collagenase IV solution for approximately 3, 5, and 7 minutes, respectively, at a perfusion speed of approximately level 6. After perfusion, the liver was excised and placed in a 10 ml EP tube containing Enzyme Buffer Solution, then stored at 4°C for later use. All livers were collected before proceeding with digestion. The Pronase E/Collagenase IV solution was pre-warmed in advance. All livers were transferred to a primary cell culture biosafety cabinet for processing. They were poured into a sterile dish, and an equal volume (10 ml) of Pronase E/Collagenase IV solution was added. The livers were minced and thoroughly dispersed, then transferred to a 50 ml EP tube. The dish was rinsed with a 1:1 mixture of Enzyme Buffer Solution and Pronase E/Collagenase IV solution, and the volume was adjusted appropriately (generally not exceeding 40 ml). A 1% volume of DNase I solution was added, and digestion was performed at 37°C, 180 rpm, for 17 minutes.The centrifuge was pre-cooled. The digested suspension was filtered through a 70 µm strainer into a 50 mL EP tube. The filtered suspension was divided into appropriate volumes, and cold GBSS B solution was added to bring the total volume to 50 mL. Centrifugation was performed at 4°C, 580g, for 10 minutes. The supernatant was discarded, leaving 10 mL of solution. Then, 120 µL of DNase I solution was added, and the pellet was resuspended. Cold GBSS B solution was added again to 50 mL, followed by centrifugation at 4°C, 580g, for 10 minutes. The supernatant was discarded, leaving 5 to 10 mL of solution, and 120 µL of DNase I solution was added for resuspension. Cold GBSS B solution was added to a final volume of 15 mL and mixed well. 30 mL of 18% Nycodenz solution was added to the cell suspension and mixed thoroughly. The mixture was aliquoted into 15 mL EP tubes (equal volumes per tube). Then, 2 mL of cold GBSS B solution was gently layered on top of each tube (first wetting the tube wall before slowly adding with a syringe). Centrifugation was performed at 4°C, 1380g, for 17 minutes (acceleration: 1, deceleration: 0). After centrifugation, the white cell layer at the interface was carefully aspirated using a Pasteur pipette. The collected cells were washed once or twice with cold GBSS B solution and centrifuged at 4°C, 580g, for 10 minutes. The cell pellet was resuspended in 3 mL of DMEM containing 20% FBS. Cell count and viability were assessed using Trypan Blue staining. The cells were then seeded at a density of 5 ×105 cells/well in a 12-well plate. After microscopic examination, the plate was gently shaken and placed in an incubator. The cells were ready for experiments after 24 hours.

### 2.2 Natural Activation Model of Mouse Hepatic Stellate Cells and Drug Administration

The isolated primary mouse hepatic stellate cells (HSCs) were allowed to adhere and cultured naturally for 3 days until spontaneous activation occurred. After activation, the medium was replaced with serum-free drug-containing medium supplemented with 25 µM Silybin (Sil), 10 µM Carvedilol (Car), or a 1:1 mixture of 50 µM Silybin and 20 µM Carvedilol. The cells were incubated for 12 hours, then harvested for qRT-PCR analysis to measure the mRNA expression levels of fibrosis-related genes Col1a1 and Col1a2.

### 2.3 qRT-PCR (reverse transcription-polymerase chain reaction)

The cell culture plate was collected, and 1 mL of RNAiso Plus was added to each well. The lysate was repeatedly pipetted using a 1 mL pipette until the solution became clear and free of cell clumps. The lysate was then transferred into a 1.5 mL EP tube. Total RNA was extracted following the instructions of the RNAiso Plus Total RNA Extraction Kit. RNA concentration and purity were quantified using a spectrophotometer. Reverse transcription was performed using an RT-PCR Kit to convert mRNA into cDNA. The reaction mixture was prepared according to the qRT-PCR Kit instructions, with a final volume of 15 µL including cDNA and reaction mix. The PCR reaction was carried out by setting the corresponding program with the following conditions of quantitative PCR reaction as follows.
Step1:95°C Pre-denaturation 1 min
Step2: PCR Reaction: 95°C, 15 s; 60°C, 15 s; 72 °C, 30 s; 40 Cycles
Step3: Melt Curve Analysis 65 °C-95 °C

### 2.4 Analysis of Compound Joint Action Index

The specific method was the same as part 2.4 of Experimental Example 3.

### 3 Experimental Results

As shown in Figure 4, on the activation model of primary hepatic stellate cells in mice, it was found that in the combined administration group of carvedilol and silybin, the mRNA levels of activated hepatic stellate cell-specific expression genes Col1a1 and Col1a2 were significantly down-regulated, and were superior to the respective single administration groups of the two drugs. The combined efficacy indexes of the two drugs were analyzed by micro-use CompuSyn software. The CI values were Col1a1 (CI=0.54) and Col1a2 (CI=0.36), which showed strong synergistic effect.

### Experimental Example 5 Investigation on Dosage Ratio of Carvedilol and Silybin Composition

### 1 Experimental Materials

The experimental materials were the same as those in Experimental Examples 1 and 2.

### 2 Experimental Methods

### 2.1 LX-2 Cell Line Culture and Transfection

The specific method was the same as part 2.1 of Experimental Example 1.

### 2.2 LX-2 Cell Administration

The carvedilol administration concentration was fixed at 10 µM, with 6:1; 4:1; 2:1; 1:1; 1:2; 1:4; 1:6; 1:8 dose concentration ratio in combination with silybin. The administration concentration of silybin was fixed at 10 µM, with 6:1. 4:1; 2:1; 1:2; 1:4; 1:6; 1:8 dose concentration ratio in combination with carvedilol.

The specific method was the same as part 2.2 of Experimental Example 3.

### 2.3 Duo-LiteTM Luciferase Assays System Kit Detection

The specific method was the same as part 2.3 of Experimental Example 1.

### 2.4 Analysis of Compound Joint Action Index

The specific method was the same as part 2.4 of Experimental Example 3.

### 3 Experimental Results

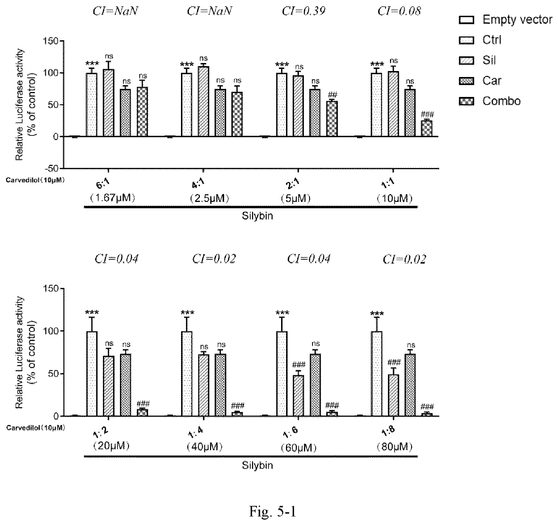

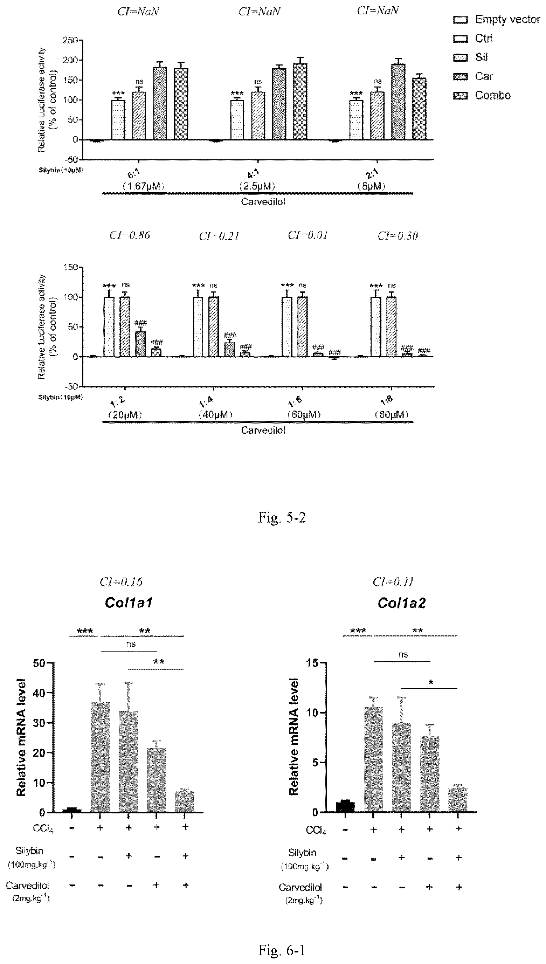

As shown in Figures 5-1 and 5-2, the results show that on activated LX-2 cells, the combination of carvedilol 10µM and silybin at 2:1, 1:1, 1:2, 1:4, 1:6, 1:8 administration concentration, the combination of silybin 10µM and carvedilol at 1:2, 1:4, 1:6, 1:8 administration concentration all show synergistic effects. In each combination, Car: 10µM, 2:1 (Sil: 5 µM; CI=0.39); 1:1 (Sil:10µM; CI=0.08); 1:2 (Sil: 20µM; CI=0.04); 1:4 (Sil: 40µM; CI=0.02); 1:6 (Sil: 60µM; CI=0.04); 1:8 (Sil:80µM; CI=0.02); Fixed Sil:10 µM, 1:2 (Car: 20 µM; CI=0.86); 1:4 (Car:40µM; CI=0.21); 1:6 (Car:60µM; CI=0.01); 1:8 (Car:80µM; CI=0.30).

### Experimental Example 6 The Effect of Carvedilol Combined with Silybin on Hepatic Fibrosis in Mice

### 1 Experimental Materials

Male C57BL/6J mice, 8 weeks old and 20-24g weight, were purchased from Beijing Vital River Animal Technology Co., Ltd.. CCl4 was purchased from Shanghai Lingfeng Chemical Reagent Co., Ltd., and mineral oil was purchased from Sigma-Aldrich (St. Louis, MO, USA).

The rest of the experimental materials were the same as Experimental Example 4.

### 2 Experimental Methods

### 2.1 Effect of Silybin (100mg/kg) in Combination with Carvedilol (2mg/kg) on Hepatic Fibrosis Induced by CCl4

After one week of acclimatization, the mice were randomly divided into five groups (n=6 per group, total 30 mice): Control group, Model group, Silybin (Sil) monotherapy group, Carvedilol (Car) monotherapy group, Sil + Car combination therapy group. Among others, the model and treatment groups received intraperitoneal injections of CCl4 (1:8 dilution in mineral oil), while the control group received an equal volume of vehicle (mineral oil). Injections were administered twice weekly at 5 mL/kg for 6 weeks. Starting from the second week of modeling, oral gavage treatment was initiated: Sil (100 mg/kg), Car (2 mg/kg), Sil + Car (100 mg/kg + 2 mg/kg). Treatments were administered once daily for 4 weeks. At the end of the treatment period, the mice were euthanized, and liver tissues were collected and preserved for further analysis.

### 2.2 Effect of Silybin (100mg/kg) in Combination with Carvedilol (2mg/kg) on BDL-induced Hepatic Fibrosis

After one week of acclimatization, the mice were randomly divided into five groups (n=6 per group, total 30 mice): Sham-operated group, BDL surgery model group (bile duct ligation-induced cholestatic liver fibrosis), Silybin (Sil) monotherapy group (100 mg/kg), Carvedilol (Car) monotherapy group (2 mg/kg), Sil + Car combination therapy group (100 mg/kg + 2 mg/kg). Among others, the model and treatment groups underwent bile duct ligation (BDL) to induce cholestatic liver fibrosis. The sham-operated group received the same surgical procedure without bile duct ligation. Starting one day post-surgery, treatments were administered via oral gavage once daily for 7 days, with the dosage of Sil 100 mg/kg, Car 2mg/kg. At the end of the treatment period, mice were euthanized, and liver tissues were harvested and preserved for subsequent analysis.

### 2.3 Effects of Silybin (75,100 mg/kg) in Combination with Carvedilol (2mg/kg) on MCD-in duced Hepatic Fibrosis (NASH)

After one week of acclimatization, the mice were randomly divided into five groups (n=6 per group, total 30 mice): MCS control group, MCD model group, Silybin (Sil) monotherapy group, Carvedilol (Car) monotherapy group, Sil+Car combination therapy group. The MCD model and treatment groups were fed with a methionine-choline deficient (MCD) high-fat diet to induce NASH-related liver fibrosis. The control group received an MCS control diet. The dietary intervention lasted for 6 weeks. Starting from week 2 of model induction, the mice received daily oral gavage of: Sil (75 or 100 mg/kg), Car (2 mg/kg). The treatment continued for 4 weeks. At the end of the treatment period, the mice were euthanized. Liver tissues were collected and preserved for subsequent analysis.

### 2.4 qRT-PCR (reverse transcription-polymerase chain reaction)

Gene to be detected: *Col1a1, Col1a2*(*Col3a1*)

The specific method was the same as part 2.3 of Experimental Example 4.

### 2.5 Pathological Analysis of Liver

A portion of liver tissues was fixed in 4% paraformaldehyde and subsequently submitted to Wuhan Guge Biotechnology Co., Ltd (Wuhan, China) for double-blind histological evaluation. The following staining analyses were performed: HE staining, Masson staining, Sirius Red staining. The collagen volume fraction (CVF) was calculated from Masson-stained sections using ImageJ software (NIH, USA) for objective quantification of fibrosis severity.

### 3 Experimental Results

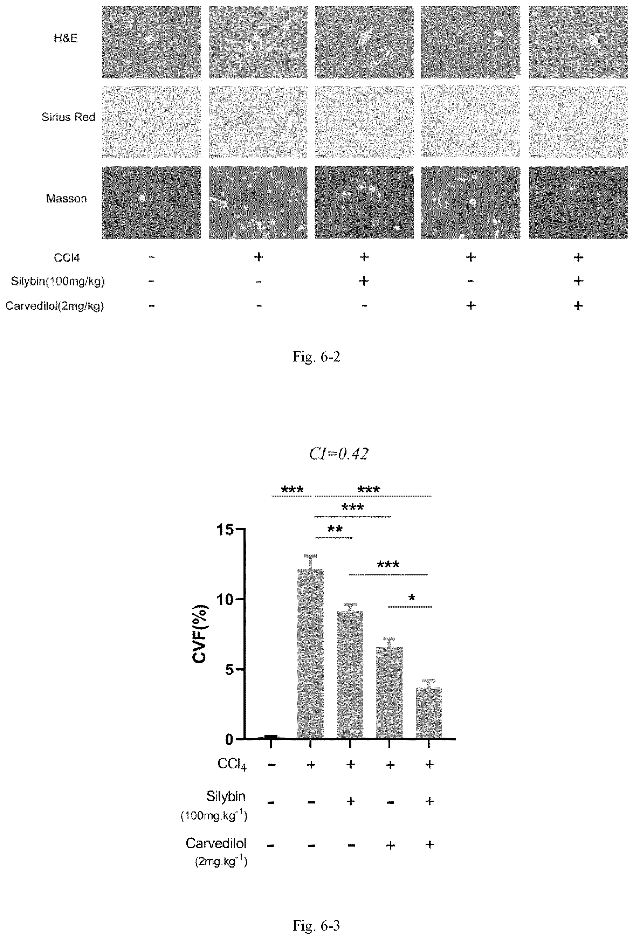

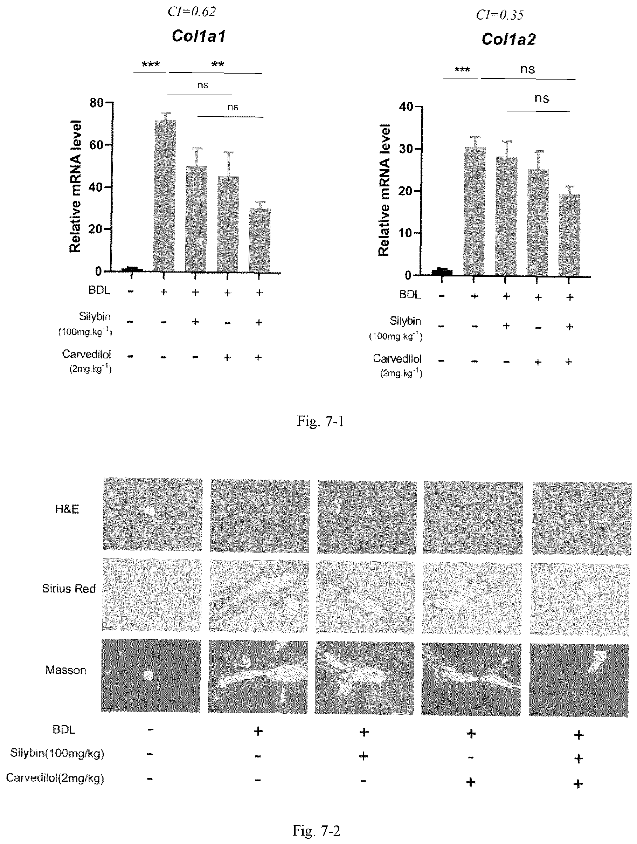

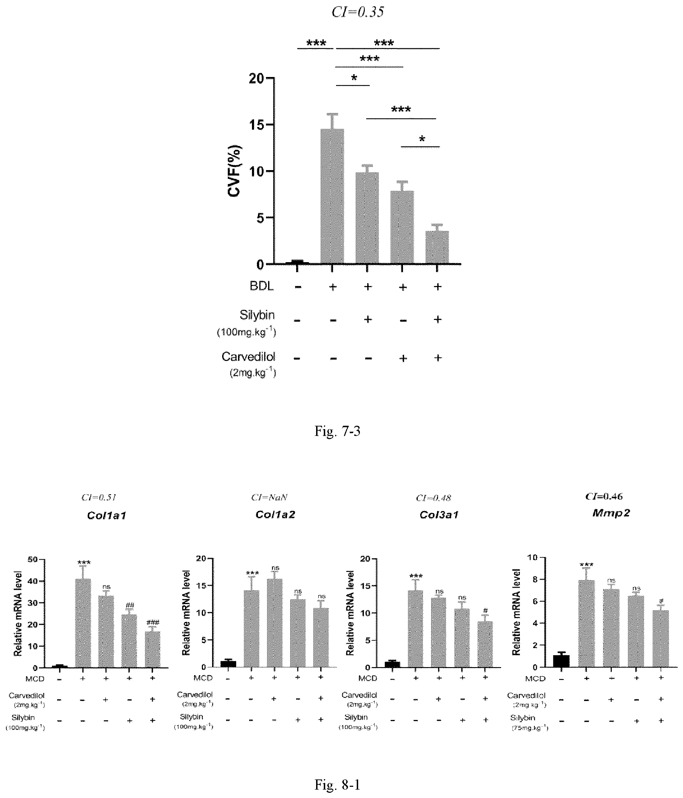

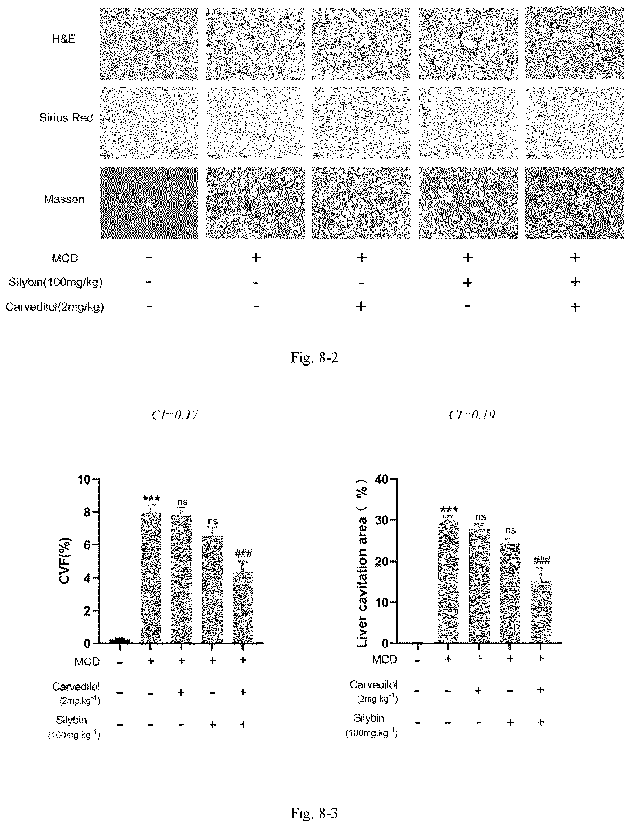

As shown in Figure 6, Figure 7 and Figure 8, according to the results of qRT-PCR experiments on the expression of liver tissue fibrosis related genes Col1a1, Col1a2(Col3a1), Mmp2 and liver pathology analysis, in the liver fiber model induced by CCl₄, BDL and MCD diets, silybin combined with carvedilol (including dosage ratios of 50:1, 100:2 and 75:2) significantly down-regulated the expression levels of Col1a1, Col1a2(Col3a1) and Mmp2 mRNA, and effectively improved the degree of hepatic fibrosis lesions in various hepatic fibrosis models. The combined efficacy of the two drugs was superior to the single efficacy of the two drugs, which was calculated according to the CI value of each index and shows a synergistic effect.

### Experimental Example 7 Investigation on Dose-dependent Pharmacological Effects of Silybin and Carvedilol Compositions with Fixed Ratios on Hepatic Fibrosis Caused by Various Factors

### 1 Experimental Materials

The experimental materials were the same as those in Experimental Example 6.

### 2 Experimental Methods

### 2.1 Dosage-dependent Pharmacological Effects of Silybin and Carvedilol Compositions with Fixed Ratios on CCl4-induced Chemical Toxic Hepatic Fibrosis

After one week of acclimatization, mice were randomly divided into eight groups (n=6 per group, total 48 mice): Control group, Model group, Obeticholic acid (OCA) 1.5 mg/kg group, OCA 5 mg/kg group, Sil 50 mg/kg + Car 1 mg/kg group, Sil 100 mg/kg + Car 2 mg/kg group, Sil 150 mg/kg + Car 3 mg/kg group, Sil 200 mg/kg + Car 4 mg/kg group, wherein model and treatment groups was administered by intraperitoneal injection of CCl4 (1:8 in mineral oil) at 5 mL/kg, and the control group received equal volumes of mineral oil vehicle, administered twice weekly for 6 weeks. Starting from week 2 of modeling, all treatments were delivered by daily oral gavage for 4 weeks. At the end of the treatment period, the mice were euthanized. Liver tissues were collected and preserved for subsequent analysis.

### 2.2 Dosage-dependent Pharmacological Effects of Silybin and Carvedilol Compositions with Fixed Ratios on BDL-induced Cholestatic Hepatic Fibrosis

After one week of acclimatization, mice were randomly divided into nine groups (n=6 per group, total 54 mice): Sham group, BDL model group, ursodeoxycholic acid (UDCA) 120 mg/kg group, Obeticholic acid (OCA) 1.5 mg/kg group, OCA 5 mg/kg group, Sil 50 mg/kg + Car 1 mg/kg group, Sil 100 mg/kg + Car 2 mg/kg group, Sil 150 mg/kg + Car 3 mg/kg group, Sil 200 mg/kg + Car 4 mg/kg group, wherein BDL model and treatment groups received bile duct ligation (BDL) to induce cholestatic hepatic fibrosis, and the sham group underwent identical surgical procedures without BDL. Starting one day post-surgery, treatments were administered by daily oral gavage for 7 days. At the end of the treatment period, the mice were euthanized. Liver tissues were collected and preserved for subsequent analysis.

### 2.3 qRT-PCR (reverse transcription-polymerase chain reaction)

Gene to be detected: *Col1a1, Col1a2*

The specific method was the same as part 2.3 of Experimental Example 4.

### 2.4 Pathological Analysis of Liver

The specific method was the same as part 2.5 of Experimental Example 6.

### 3 Experimental Results

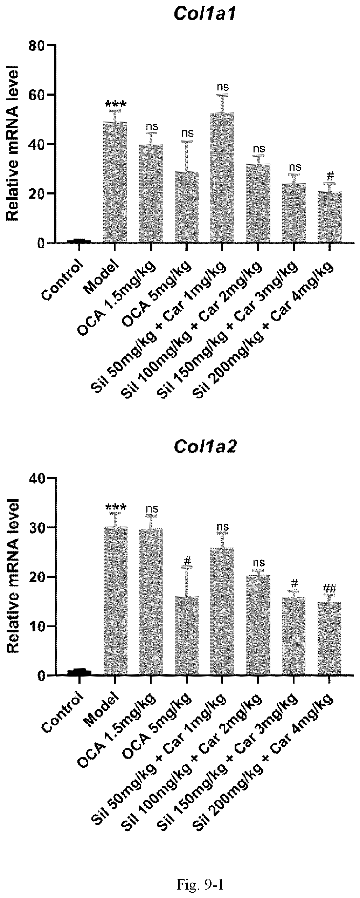

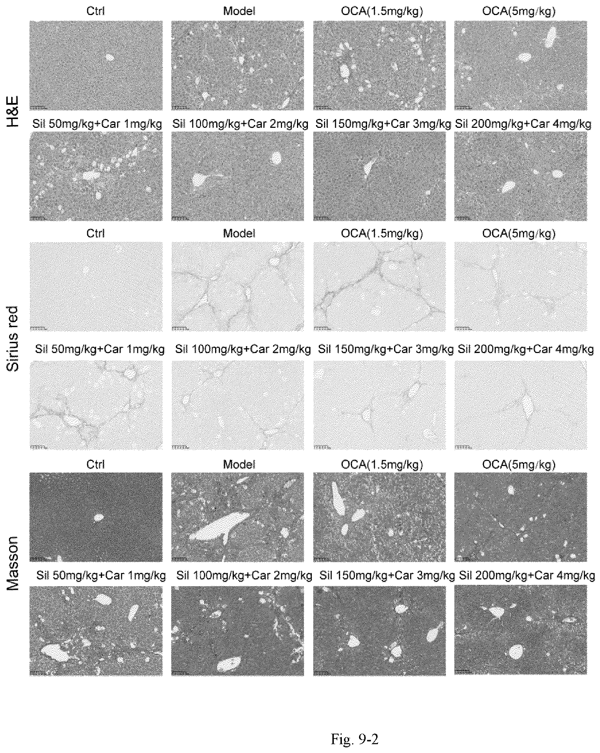

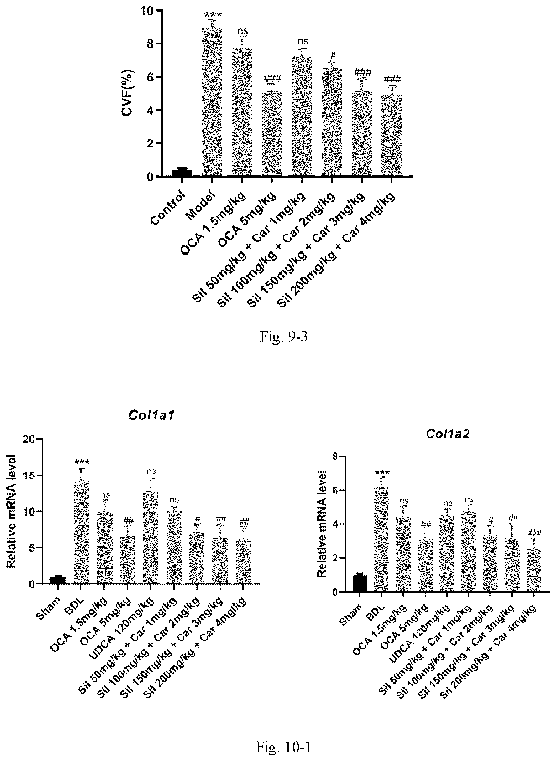

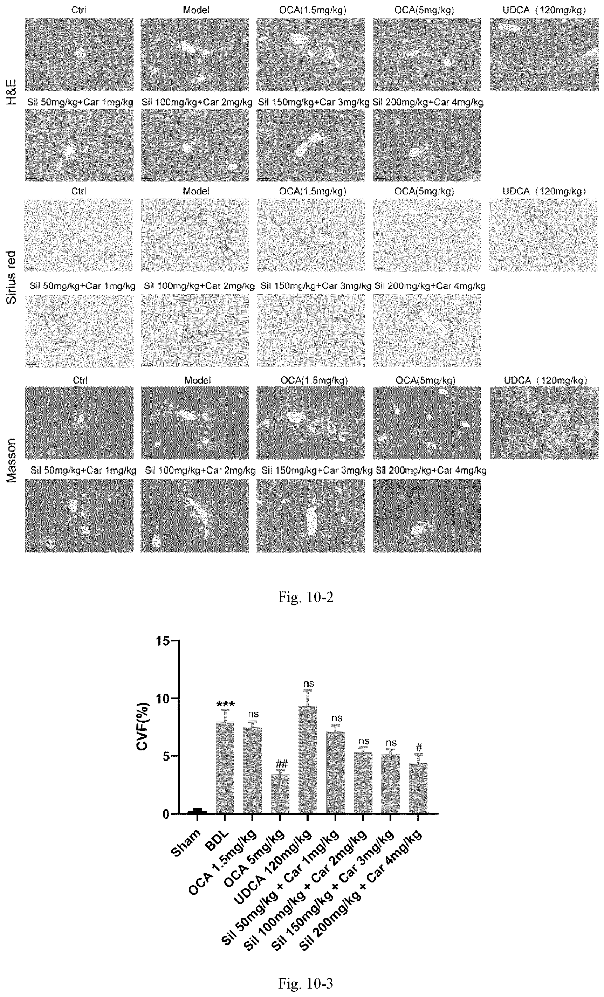

As shown in Figures 9 and 10, according to the results of qRT-PCR experiment on the expression of liver tissue fibrosis related genes Col1a1 and Col1a2 and liver pathology analysis, in the liver fiberosis model induced by CCl4 and BDL, the combination of silybin and carvedilol at a fixed dose ratio significantly down-regulates the expression level of Col1a1 and Col1a2 mRNA, effectively improves the degree of hepatic fibrosis lesion in the two hepatic fibrosis models, and shows a definite dose-dependent tolerance. For CCl4-induced chemical toxic hepatic fibrosis, the anti-fibrosis efficacy of the composition with a fixed ratio of 200mg/kg silybin and 4mg/kg carvedilol was better than 1.5mg/kg OCA (Obeticholic acid) and was equivalent to that of 5mg/kg OCA. For BDL-induced cholestatic hepatic fibrosis, the anti-fibrosis efficacy of a composition with a fixed ratio of 200mg/kg silybin and 4mg/kg carvedilol was better than 120mg/kg positive drug UDCA and 1.5mg/kg OCA, and was equivalent to 5mg/kg OCA.

### Example 1

A silybin carvedilol composition is shown below

| Excipients Composition | Quantity (mg) | Ratio (%) |
|---|---|---|
| Carvedilol | 3 | 1.2 |
| Silybin | 35 | 14 |
| Lactose | 97 | 38.8 |
| Sodium carboxymethyl starch | 20 | 8 |
| Talc | 30 | 12 |
| Soybean phospholipid | 65 | 26 |

The preparation method comprises dissolving silybin, carvedilol and soybean phospholipid in anhydrous ethanol, mixing with lactose and sodium carboxymethyl starch as binder, granulating, drying, granulating, mixing with talc, and encapsulating.

### Example 2

A silybin carvedilol composition is shown below

| Excipients Composition | Quantity (mg) |
|---|---|
| Carvedilol | 1 |
| Silybin | 50 |
| Lactose | 97 |
| Sodium carboxymethyl starch | 20 |
| Talc | 30 |
| Soybean phospholipid | 65 |

The preparation method comprises dissolving silybin, carvedilol and soybean phospholipid in anhydrous ethanol, mixing with lactose and sodium carboxymethyl starch as binder, granulating, drying, granulating, mixing with talc, and encapsulating.

## Claims

1. A silybin-containing composition, comprising silybin and carvedilol as active ingredients.

2. The silybin-containing composition according to claim 1, comprising a) silybin and carvedilol as active ingredients; and b) a pharmaceutically acceptable excipient suitable for preparaing a solid oral dosage form; wherein the mass ratio of silybin and carvedilol is 0.1 to 200: 1.

3. The silybin-containing composition according to claim 2, wherein the mass ratio of silybin and carvedilol is 1 to 50:1.

4. The silybin-containing composition according to claim 2, wherein the mass ratio of silybin and carvedilol is 2 to 50:1.

5. The silybin-containing composition according to claim 2, wherein the mass ratio of silybin and carvedilol is 1 to 8:1.

6. The silybin-containing composition according to claim 2, wherein the mass ratio of silybin and carvedilol is 2 to 8:1.

7. The silybin-containing composition according to claim 2, wherein the mass ratio of silybin and carvedilol is 37.5 to 50:1.

8. The silybin-containing composition according to claim 1, wherein the mass ratio of silybin and carvedilol is 50:1.

9. The silybin-containing composition according to claim 1, wherein the carvedilol includes the pharmaceutically acceptable salt formed with organic acid or inorganic acid.

10. The silybin-containing composition according to claim 9, wherein the organic acid includes oxalic acid, fumaric acid, benzoic acid and mandelic acid; and the inorganic acid includes phosphoric acid.

11. A silybin-containing composition, comprising a) silybin; and b) a non-steroidal anti-inflammatory drug, an angiotensin inhibitor, a calcium antagonist, a glucocorticoid drug, or a diuretic.

12. The silybin-containing composition according to claim 11, wherein the non-steroidal anti-inflammatory drug is selected from aceclofenac or clobetasol propionate; the angiotensin inhibitor is valsartan; the calcium antagonist is selected from nicardipine, manidipine, amlodipine, or cilnidipine; the glucocorticoid drug is budesonide; the diuretic is selected from tamsulosin or D-mannitol.

13. A use of the silybin-containing composition according to any one of claims 1 to 12 in preparation of medicine for the treatment of hepatic fibrosis.

14. A use of the silybin-containing composition according to any one of claims 1 to 12 in preparation of medicine for the treatment of chronic liver diseases.

15. The use according to claim 13, wherein the use is the use in preparation of medicine for the treatment of chronic liver diseases, and the hepatic fibrosis caused thereby.

16. The use according to claim 13, wherein the use is the use in preparation of medicine for the treatment of chemical liver damage, cholestatic liver disease and non-alcoholic steatohepatitis; and the hepatic fibrosis caused thereby.
